# EUROPEAN PATENT APPLICATION

(11) **EP 1 985 312 A1**
(43) Date of publication of application: **29.10.2008**
(21) Application number: 07106928.0
(22) Date of filing: 25.04.2007
(51) Int. Cl.: A61K 49/08, A61K 49/10, A61K 49/18, C07C 49/167, C07C 49/24, C07C 49/92, C07F 5/00, A61B 5/055

(54) **T1 reduction in 19F magnetic resonance imaging (MRI)**

(71) Applicant: Koninklijke Philips Electronics N.V., 5621 BA Eindhoven (NL)
(72) Inventor: The designation of the inventor has not yet been filed
(74) Representative: Van Velzen, Maaike Mathilde

(57) **Abstract**

Paramagnetic complexes are provided which are designed for the purpose of shortening the T1 relaxation time of fluorinated contrast agents in ¹⁹F Magnetic Resonance Imaging. The complexes are miscible with the contrast agents. Preferably, the complexes are included in perfluorinated nanoparticle ¹⁹F MRI contrast agents.

## Description

### FIELD OF THE INVENTION

The invention relates to Magnetic Resonance Imaging (MRI) based on the detection of non-aqueous ¹⁹F contrast agents. More particularly, the invention relates to adjuvants which aid in increasing the sensitivity of ¹⁹F-MRI when based on nanoparticles of fluorinated, preferably perfluorinated organic, compounds.

### BACKGROUND OF THE INVENTION

Morawski et al., Magn. Reson. Med. 52, 1255 (2004), describe the quantitative molecular imaging of fibrin, using a non-aqueous ¹⁹F contrast agent, viz. ligand-targeted perfluoro nanoparticles. Morawsi describes the use of ¹H MRI as well as ¹⁹F MRI. Magnetic Resonance Imaging based on ¹⁹F instead of ¹H opens up new diagnostic possibilities. Since ¹⁹F does not naturally occur in the body, ¹⁹F MRI will necessarily be based on the use of added ¹⁹F contrast agents. As only signals from these agents are detected, ¹⁹F MRI has a high specificity.

A drawback to ¹⁹F MRI, however, is the low sensitivity (as reflected in a low signal-noise ratio), which can be attributed to a long T1 relaxation time, as briefly explained hereinafter.

In MRI, a strong magnetic field is used to align certain nuclei (protons in ¹H MRI and ¹⁹F nuclei in ¹⁹F MRI), which results in a net magnetic field. After excitation by a radio frequency (RF) pulse, this magnetization will generate an RF signal that can be detected. This RF signal is characterized by a frequency that is related to the magnetic field strength, which allows the use of magnetic field gradients to encode the spatial information needed to reconstruct an image from the detected signals. Two relaxation times from the excited state (T1 and T2) are taken into account, T2 related to the echo time, and T1 to the repetition time (TR) of the MRI sequence.

If T1 is too long in relation to TR, a loss of signal will occur, and hence a loss of sensitivity, due to so-called T1 saturation. In other words: the percentage of nuclei that has gone through relaxation and can be excited by a next RF pulse diminishes. In ¹⁹F MRI there is need for shortening the T1 of the ¹⁹F contrast agents used.

T1 shortening exists in ¹H MRI, where contrast agents are used having permanent magnetic dipoles, which influences the relaxation process of the nearby water protons. In ¹H MRI, paramagnetic gadolinium (Gd) complexes are employed to shorten the T1 of water or at least shorten T1 more strongly than T2. This effect on the surrounding water creates a difference between the ¹H signal from sites where the targeted particles are present, and sites where they are not.

Thus, the ¹H MR signal originating from areas with an increased gadolinium concentration has a significantly shorter T1 and yields a higher signal intensity when imaged by most MRI sequences.

Morawski, in detecting the ligand-targeted perfluoro nanoparticles by ¹H NMR, utilizes this concept by having chelated gadolinium atoms situated in the bounding lipid of the nanoparticles. This refers to water-soluble gadolinium compounds which penetrate into the surrounding water, thereby locally enhancing the ¹H relaxation rates (i.e. shortening T1 and T2).

When using ¹H MRI to detect ligand-targeted perfluorinated particles, the only way to detect the contrast agent is by influencing the relaxation times of the surrounding water. By using ¹⁹F MRI as well, Morawski also relates to the straightforward, direct detection of the ¹⁹F signal of the perfluorinated particles, without any such added measures as the use of gadolinium.

Ratner, et al., Invest. Radiology 23, 224 (1989) describes the use of aqueous based Gd agents, for the T1 reduction of an inorganic, aqueous-based ¹⁹F contrast agent, viz. an aqueous sodium fluoride solution. Lee et al., Journal of Magnetic Resonance Imaging, 1994, no. 4/4, pages 609-613, provides a similar disclosure. These disclosures do not pertain to the detection of non-aqueous ¹⁹F contrast agents.

### SUMMARY OF THE INVENTION

The invention seeks to provide agents which serve to aid in T1 reduction of ¹⁹F in non-aqueous ¹⁹F contrast agents, with the objective to enhance sensitivity of ¹⁹F MRI. The invention particularly seeks to achieve such T1 reduction in fluorinated organic compounds, and more particularly in highly fluorinated and perfluorinated compounds, when used as MRI contrast agents in ¹⁹F MRI,

With reference to the above objectives, the invention, in one aspect, is a paramagnetic complex comprising at least one paramagnetic metal having at least one coordination site complexed with a fluorophilic ligand.

In another aspect, the invention provides for a method of detecting ¹⁹F contrast agents using ¹⁹F MRI, wherein paramagnetic agents are used which are soluble in, or miscible with, non-aqueous ¹⁹F contrast agents, and preferably in organic, highly-fluorinated and perfluorinated liquids. In yet another aspect, the invention provides for the use of fluorophilic paramagnetic agents in ¹⁹F MRI, particularly for the purpose of reducing T1 of ¹⁹F atoms.

### DETAILED DESCRIPTION OF THE INVENTION

The invention particularly relates to ¹⁹F MRI based on the use of non-aqueous ¹⁹F contrast agents. In the context of the invention, this term is used to indicate contrast-agents for MRI which are at least insufficiently water-soluble to be introduced in the form of an aqueous solution or which, if intrinsically water-soluble, are introduced into the human or animal body in such a way so as to form a phase separate from body fluids.

Aqueous solutions of fluorine based contrast agents, while useful for scientific investigation, are less suitable for use in practice. In practice, it is desired to use fluorinated compounds which introduce as a high a concentration of fluorine as possible and which can be used in a non-toxic way.

The contrast agents will be generally used to visualise a bio-molecular signature which is specific for a desease. For example, particular proteins will be overexpressed on the surface of cancer cell compared to normal cells. Here we aim at performing molecular imaging by MRI. One of the major issues for Molecular Imaging by MRI is the sensitivity. This can be overcome by using particulate agents. The advantage of the particles is that locally high concentrations of MR detectable components can be realized.

Good examples of preferred non-aqueous, highly fluorinated ¹⁹F contrast agents are perfluorinated nanoparticles. Such nanoparticles are described, e.g., in the above-identified paper by Morawski et al. Other disclosures of suitable nanoparticles include Mulder et al. in NMR Biomed. 2006; 19:142-164.

The invention includes the use of paramagnetic agents having at least one ligand which is fluorophilic. This term is used in analogy with the well-known term "hydrophilic" and denotes a tendency of the ligand to mix with fluorinated agents. The fluorophilic ligand is coordinated to a paramagnetic metal, resulting in paramagnetic agent with high fluorophilicity. Preferably, the fluorophilicity of the agent is such that it is miscible with the above-described fluorinated contrast agents and, more preferably, soluble in highly fluorinated liquids, such as the perfluorinated liquids used in the emulsions contained in the preferred nanoparticles.

This results in a marked difference over the use of paramagnetic agents to reduce T1 in ¹H NMR. In the latter case, the water molecules in fact occupy at least one of the coordination sites of the paramagnetic metal. Depending of the chelate, the water can even interact with more coordinations sites of the metal ion. As a result, the T1 reductivity of the paramagnetic metal is believed to be based on so-called inner-sphere interaction. In the case of paramagnetic agents for fluorinated contrast agents, the paramagnetic metal will not coordinate with fluor and will thus be intrinsically turned away from the fluorinated phase. Or, as may be preferred in some applications, the metal will be shielded from the fluorinated phase by having all of the coordination sites of the metal occupied with ligands that are miscible with the perfluorinated agent. Without any of the believed theories being considered as binding, the T1 reduction of ¹⁹F as realized with the paramagnetic agents of the invention, can unexpectedly be explained with reference to so-called second sphere and outer-sphere interactions.

The paramagnetic metal can be any metal having paramagnetic properties. Paramagnetic metals are known to the skilled person, and do not require elucidation here. E.g., early and late transition metals, explicitely including chromium, manganese, iron, as well as lanthanides, such as gadolinium, europium, dysprosium, holmium, erbium, thulium, ytterbium, and in general ferromagnetic metals above their Curie temperature. The term "metal" denotes neutral metal ions or positively or negatively charged metal ions, or clusters or alloys comprising any combination of those. Many of these metals shorten both T1 and T2. Preferred are those paramagnetic metals which result in the greatest difference of T1 reduction over T2 reduction, of which gadolinium is the most preferred example.

Preferably, the fluorophilic T1 contrast agents of the invention are paramagnetic metal complexes having a good solubility in highly fluorinated liquids, such as perfluorooctylbromide (PFOB). Such solubility is best achieved by using coordinating ligands that themselves contain fluorinated alkyl groups. E.g. β-hydroxyalkenones with the general structure 1 below are highly suitable ligands for the coordination to lanthanide and transition metal ions. In Formula 1 the R groups are as discussed below. Formulae 2 and 3 depict metal complexes and metal complexes with co-ligands, as discussed hereinbelow.

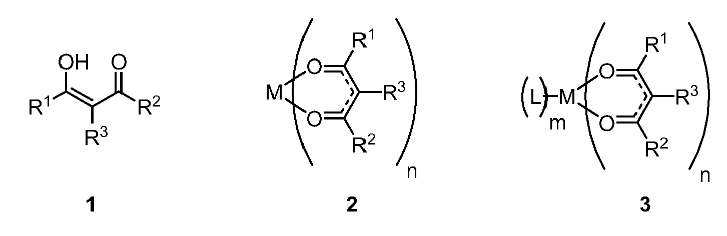

With reference to Formula 1, R, R¹, R², and R³ independent from each other represent hydrocarbon or halogenated hydrocarbon containing groups. The term hydrocarbon as used herein can denote straight-chained, branched and cyclic aliphatic and aromatic groups, and can typically include alkyl, alkenyl, alkynyl, cycloalkyl, cycloalkylalkyl, aryl, arylalkyl, arylalkenyl and arylalkynyl. The term "hydrocarbon containing group" also allows for the presence of atoms other than carbon and hydrogen, typically for example, silicon. The term "hydrocarbon containing group" particularly allows for the presence of the halogen atoms F, Cl, Br, I.

The term hydrocarbon as explained above can denote straight-chained, branched and cyclic aliphatic and aromatic groups, and can typically include alkyl, alkenyl, alkynyl, cycloalkyl, cycloalkylalkyl, aryl, arylalkyl, arylalkenyl and arylalkynyl. Suitably, the hydrocarbon groups can contain up to 35 carbon atoms, typically up to 30 carbon atoms, and more typically up to 20 carbon atoms. Corresponding halogenated hydrocarbons can also be employed, especially halogenated and particularly fluorinated hydrocarbons are preferred. In a preferred case where R represents a fluorinated alkyl group, this can be represented by the general formula F(CF₂)ₖ(CH₂)₁, where k is typically an integer having a value between 1 and 30 and 1 is an integer having a value of between 0 and 6. More preferably, k is an integer of between 1 and 10. It is of course recognized that although the above are given as preferred ranges for the values of k and 1, the particular choice of k and 1 will depend on the composition of the formulation of the fluorinated liquid. It will also be appreciated that the term "hydrocarbon containing group" also allows for the presence of atoms other than carbon and hydrogen, typically Si, as explained above.

The above hydrocarbon groups can also be further substituted by halogenated or non-halogenated substituents well known in the art, such as C₁₋₆alkyl, phenyl, C₁-₆haloalkyl, hydroxy, C₁₋₆alkoxy, C₁₋₆alkoxyalkyl, C₁₋₆alkoxyC₁₋₆alkoxy, aryloxy, keto, C₂-₆alkoxycarbonyl, C₂₋₆alkoxycarbonylC₁₋₆alkyl, C₂₋₆alkylcarbonyloxy, arylcarbonyloxy, arylcarbonyl, amino, mono- or di- (C₁₋₆)alkylamino, or any other suitable substituents known in the art.

The preferred agents show a sufficient solubility in highly fluorinated liquids, such as perfluorooctylbromide (PFOB). Such solubility is best achieved by using coordinating ligands that themselves contain fluorinated alkyl groups. β-hydroxyalkenones with the general structure 1 are highly suitable ligands for the coordination to lanthanide and transition metal ions. The groups Rⁿ are most preferably fluorinated alkyl or aryl groups. Nevertheless non-fluorinated alkyl groups or hydrogen atoms are also suitable provided that the resulting complexes still exhibit a sufficient solubility in fluorinated liquids (for a further definition of R see below).

The solubility of metal complexes in highly hydrophobic fluorinated liquids is also influenced by the overall charge of the compounds. Preferably the metal complex bears no overall charge. This entails that the positive charge of the encapsulated metal ion is preferably compensated by the negative charge of the ligand groups. Metal complexes 2 are preferred examples of such neutral metal complexes. In Formula 2 the R groups are as defined above, n is an integer of from 1 to 4, and M is the paramagnetic metal. The n coordinated ligands may be identical or may have a different formula. In the latter case n different bidentate 1,3-dionates would be bound to the central metal. If the metal ion is a tripositive lanthanide ion, the coordination of three (n = 3) negatively charged 1,3-dionate derivatives results in an overall neutral complex.

We have found that the neutral complex comprising three 6,6,7,7,8,8,8-heptafluoro-2,2-dimethyl-3,5-octanedionate monoanionic ligands (fod⁻, M = Gd(III), R¹ = (CF₂)₂CF₃, R² = C(CH₃)₃, R³ = H) coordinated to one gadolinium(III) metal ion ([Gd(fod)₃]⁰) can be used to reduce the T1 relaxation time of PFOB by more than 20-fold at a metal complex concentration of about 10 mM. Higher concentrations result in even short T1 values.

The T1 relaxation effect primarily results from second and outer sphere interactions between the metal center and the fluorinated molecules. For an optimization of these interactions, it is preferred to have a short distance between the metal center and the fluorinated molecules. Thus, preferred ligands have a small bulkiness and allow for good intercalation of the fluorinated molecules. Short perfluoroalkyl groups R in compounds 1-3 fulfil these requirements, with short indicating C₁₋₁₀ and preferably C₁₋₆.

Additional co-ligands L may be coordinated to the metal center (compounds of general formula 3). In formula 3, the R groups are as defined above, n and m, each independently, are integers of from 1 to 4, with M being the paramagnetic metal, and L denoting said co-ligand.

Such co-ligands may further increase the solubility in the hydrophobic liquid or determine the distribution of the metal complex in the volume of the liquid. An anisotropic distribution may be advantageous for certain applications. The formation of ordered structures, such as bi- or micelles is explicitly not excluded. Appropriate co-ligands are mono-, bi-, or tridentate ligands. If *n* is chosen such, as to compensate for the charge of the metal ion, the co-ligands should be uncharged. Non-limiting examples of such uncharged co-ligands L are fluorinated or non-fluorinated alkylalcohols, -amines, ketones, -phosphine oxides,-sulfoxides, or -sulfondioxides. Examples of bidentate co-ligands are fluorinated or non-fluorinated 2,2'-bipyridines, phenanthrolines, and their derivatives. Difunctional fluorinated or non-fluorinated alkylalcohols, -amines, -ketones, -phosphine oxides, - sulfoxides, or -sulfondioxides may also be suitable co-ligands. Terpyridines or 1,3,5-triketones are examples of neutral tridentate co-ligands.

Charged co-ligands L may be advantageous as well, particularly if *n* is chosen too small to compensate for the charge of the metal ion, so that in the presence of L a charge-neutral complex can be obtained. Suitable negatively charged co-ligands include halogenides, and fluorinated or non-fluorinated alkylalcoholates, -carboxylates, -hydroxamates, - sulfinates, -sulfonates, -phospinates, or -phosponates.

If the charge of the central metal ion is overcompensated by the negative charge of the ligands, positively charged counter ions must be chosen such that the solubility of the complex in fluorinated solvents is ensured. Such hydrophobic cations include fluorinated or non-fluorinated tetraalkyl- or tetraarylammonium, -phosphonium, or- arsonium ions, or fluorinated or non-fluorinated alkyl- or arylpyridinium ions.

If the charge of the metal ion is incompletely compensated by the negative charge of the ligands, negatively charged counter ions have to be chosen such that the solubility of the complex in fluorinated solvents is ensured. Examples of such hydrophobic anions are tetraphenylborat or derivatives thereof bearing fluoro, fluoroalkyl, or fluoroaryl substituents.

Macrocyclic ligands are further preferred ligands. The introduction of perfluorinated alkyl- or aryl-substituents can increase the solubility of the resulting metal complexes sufficiently to make them useful in the context of this invention. Non-limiting examples of such ligands have the structures given below.

These structures serve as examples of macrocyclic ligands for the formation of metal complexes [ML]^{z-}(z = 0,1). These complexes may contain further co-ligands. R groups (R¹, R²) independent from each other are preferably (per)fluorinated alkyl or aryl groups, which optionally can be linked to the ligand backbone via functional groups, such as ether or amide groups or other linkers. Preferred values for n, m, and p are independent from each other n, m, p = 1-3, q = 1,2.

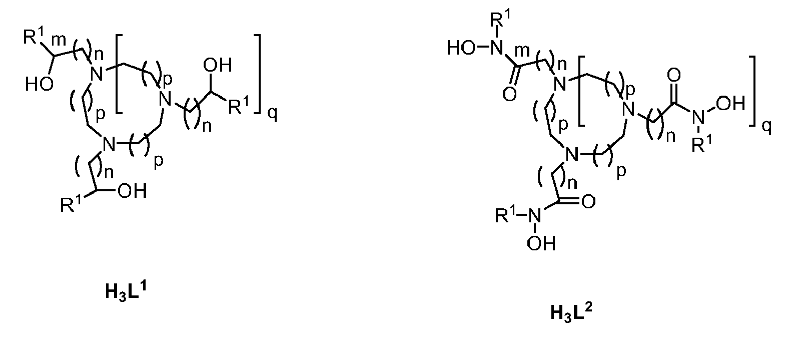

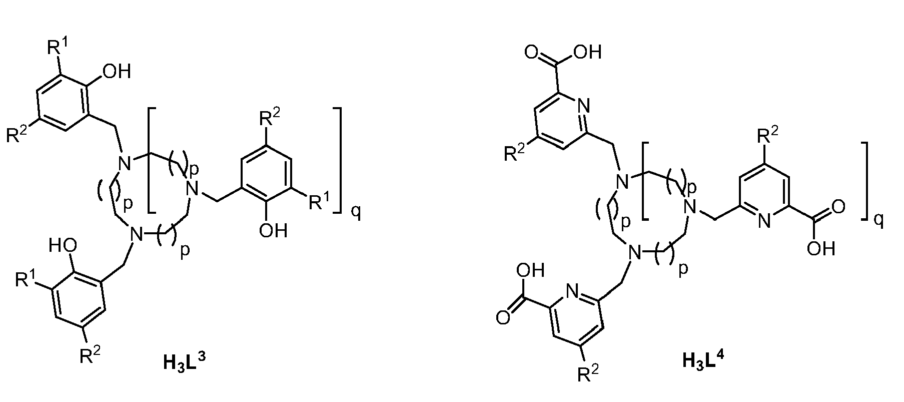

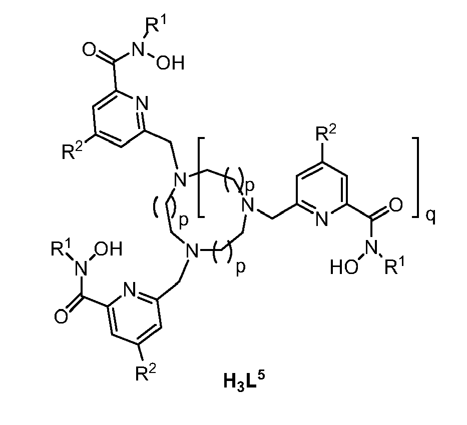

The invention also relates to the use of a paramagnetic complex as described hereinbefore as an adjuvant to reduce, in the course of a ¹⁹F Magnetic Resonance Imaging method aiming at the detection of a of a non-aqueous ¹⁹F contrast agent, the T1 relaxation time of the contrast agent.

In a preferred embodiment, the adjuvant is integrated with the contrast agent, which can be realized well in the case of nanoparticles comprising an encapsulated fluorinated liquid. By virtue of its fluorophilicity, the paramagnetic complex is dissolved in, or mixed into, the fluorinated liquid.

The invention, in another aspect, also relates to the resulting nanoparticles, which can serve as 19F MRI contrast agent with a shortened T1 relaxation time.

Preferred nanoparticle contrast agents are based on a perfluorocarbon core, e.g. perfluoro-octylbromide (PFOB), perfluoro-crown ethers (PFCE), or other perfluorinated organic compounds and a (phospho)lipid layer encapsulating the core (i.e. a shell). The layer has two functions; to stabilize the particle and to allow for interaction with ligands used for targeted binding. The size of these particles is generally in the nanometer range (50-500 nm). One could also use particles with a shell made from polymers, which are filled with a perfluoro-compound. E.g. a preferred type of such particles as can be used in the invention, and a method of making them, is described in WO 2006/046200.

To the outer hull of any of these particles the ligands for specific targeted binding can be attached. This can be done in various known manners, inter alia through covalent bonding (e.g. to a polymeric outer hull), by having hydrophobic tails penetrate into the surface of the particle (e.g. in the case of a phosholipid surface), or by using e.g. biotine - streptavidine linking.

The use of the nanoparticles according to the invention is not limited to any particular target. On the other hand, the improved 19F MRI method which results from using the complexes, and resulting nanoparticles, of the invention, are believed to enable improvements in relation to such disorders as require improvement in the detection thereof. Thus, the invention is believed to be particularly useful in the detection of tumors such as in colorectal cancer, e.g. based on CEA (carcinoembryonic antigen) or MUC1 as biomarkers, and of cardiovascular disorders such as lead to the presence of vulnerable plaques, for which e.g. fibrine is a biomarker.

The nanoparticles include in their core a paramagnetic complex in accordance with the invention, as described hereinbefore. The incorporation of the complexes according to the invention in such nanoparticles, can be done in a straightforward manner by mixing a desired quantity of the complex with a fluorinated, preferably perfluorinated liquid, before said liquid is further processed into filled nanoparticles, e.g. such as described in the aforementioned Morawski and Mulder references.

It is to be understood that the invention is not limited to the embodiments and formulae as described hereinbefore. It is also to be understood that in the claims the word "comprising" does not exclude other elements or steps. Where an indefinite or definite article is used when referring to a singular noun e.g. "a" or "an", "the", this includes a plural of that noun unless something else is specifically stated.

The invention will be illustrated with reference to the following, unlimitative Example and the accompanying Figure.

### FIGURE

The Figure shows, in the top part, an MRI scan, i.e. an image, of three vials as described in the Example. Directly underneath the depicted scan, the Figure shows, with the depicted results aligned with the depicted vials, a graphic representation of the signal intensity observed.

### EXAMPLE

0.10 g (0.10 mmol) of gadolinium tris(6,6,7,7,8,8,8-heptafluoro-2,2-dimethyl-3,5-octanedionate) (Gd(fod)₃) were dissolved in 600 mL of perfluorooctylbromide (PFOB). Aliquots of this 0.16-molar (8.6 w%) solution was further diluted with PFOB to obtain a series of solutions with different Gd(fod)₃ concentrations:

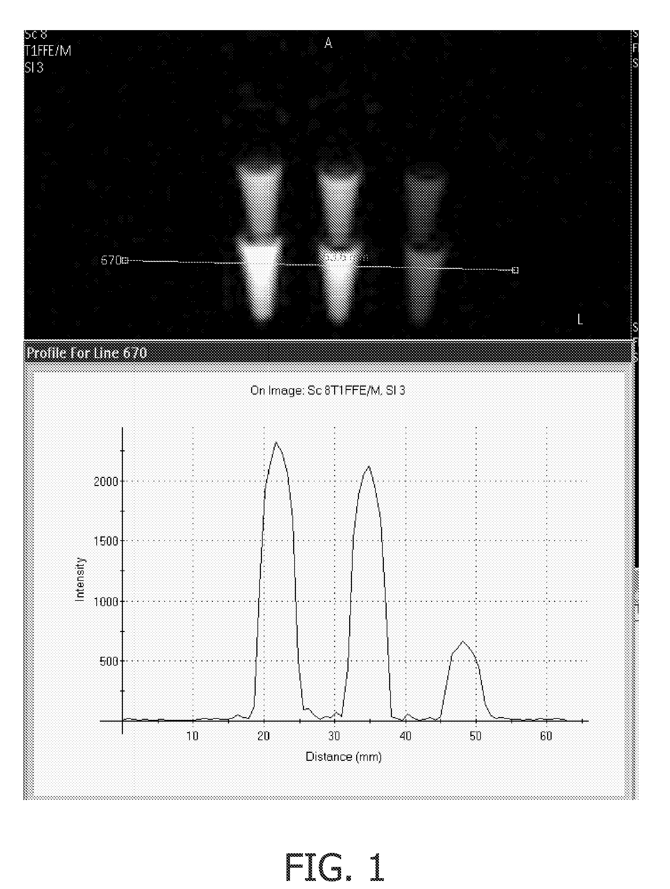

The vials shown in Figure 1, top part, contain, from left to right

| No. | [Gd(fod)₃] | mass conc. | T1 |
|---|---|---|---|
| 1 | 0.01 M | 0.55 w% | 57 ms |
| 2 | 0.0025 M | 0.14 w% | 200 ms |
| 3 | 0.0M | pure PFOB | 1300 ms |

Figure 1 shows that with the used T1-weigthed FFE (Fast Field Echo) scan the signal intensity increases with by a factor of 4 for the 0.01 M solution. Also indicated in Figure 1 are the T1 values determined at 3T, using a spectroscopic T1 sequence. The T1-relaxivity (r1) is approx 1.6 mM-1s-1, this is lower than for 1H and Gd-DPTA (4 mM-1s-1), indicating that the relaxivity for the 19F is predominantly second and outer sphere.

## Claims

1. A paramagnetic complex comprising a paramagnetic metal having at least one of its coordination sites complexed with a fluorophilic ligand.

2. A paramagnetic complex according to claim 1, wherein all of the metal's coordination sites are complexed with a fluorophilicligand.

3. A paramagnetic complex according to claim 1 or 2, wherein the ligand is a fluorinated hydrocarbon.

4. A paramagnetic complex according to any one of the preceding claims, wherein the fluorophilic ligand satisfies formula 1 on the formula sheet, wherein R, R¹, R², and R³ independent from each other represent hydrocarbon or halogenated hydrocarbon containing groups.

5. A paramagnetic complex according to any one of the preceding claims, wherein the paramagnetic metal is gadolinium.

6. A paramagnetic complex according to any one of the preceding claims, comprising short chain, C₁₋₁₀ fluorophilic ligands.

7. The use of a paramagnetic complex according to any one of the preceding claims as an adjuvant in the course of a ¹⁹F Magnetic Resonance Imaging method aiming at the detection of a of a non-aqueous ¹⁹F contrast agent, to reduce the T1 relaxation time of the contrast agent.

8. A fluorinated contrast agent for use in ¹⁹F Magnetic Resonance Imaging in the form of a nanoparticle comprising an encapsulated fluorinated liquid, wherein the fluorinated liquid comprises a paramagnetic complex according to any one of the claims 1-6.

9. A nanoparticle according to claim 8, with a ligand for targeted binding attached on the outside of the particle.

10. A nanoparticle according to claim 9, wherein the ligand is targeted for the detection of colorectal cancer.

11. A nanoparticle according to claim 9, wherein the ligand is targeted for the detection of vulnerable plaques.

12. A diagnostic method comprising the detection of targeted nanoparticles according to any one of claims 9-11 in a ¹⁹F Magnetic Resonance Imaging method, wherein the nanoparticles are contained in a human or animal subject.
